# EUROPEAN PATENT APPLICATION

(11) **EP 4 559 463 A1**
(43) Date of publication of application: **28.05.2025**
(21) Application number: 23383201.3
(22) Date of filing: 23.11.2023
(51) Int. Cl.: A61K 31/4152, A61P 21/00, A61P 25/28

(54) **EDARAVONE OR ITS SALTS FOR USE IN THE TREATMENT OF OXIDATIVE STRESS-MEDIATED NEURODEGENERATIVE DISORDERS**

(71) Applicant: Ferrer Internacional, S.A., 08173 St. Cugat del Vallès (ES); Treeway TW001 B.V., 5026 SK Tilburg (NL)
(72) Inventor: Tarragó Asamara, Cristina, 08028 Barcelona (ES); Álvarez Ávila, Lubia, 08028 Barcelona (ES)
(74) Representative: Ferrer Internacional S.A.

(57) **Abstract**

Edaravone or a pharmaceutically acceptable salt thereof is disclosed for use in the treatment of an oxidative stress-mediated neurodegenerative disorder, wherein the treatment comprises orally or gastrically administering any of them to human subjects in need thereof once daily in the morning, at a daily dose ranging from 80 to 120 mg, during an uninterrupted period of at least 1 month, wherein the human subjects have fasted for at least 2 hours before administration.

## Description

### Technical Field

The present invention relates to the therapeutic use of edaravone or its salts in human patients in oxidative stress-mediated neurodegenerative disorders such as Amyotrophic Lateral Sclerosis (ALS), as well as to the use of edaravone or its salts in combination with other drugs such as riluzole.

### Background Art

Oxidative stress is produced by elevated levels of reactive oxygen species (ROS) when the biological system is not able to detoxify reactive intermediates such as peroxides and free radicals that cause damage to lipids, proteins and DNA, nor to repair the resulting damage.

Impairments associated with oxidative stress are believed to contribute, among others, to neurodegenerative diseases, such as Amyotrophic Lateral Sclerosis, Multiple Sclerosis, Parkinson's disease, Alzheimer's disease, and Huntington's disease.

Amyotrophic lateral sclerosis (ALS) is a rapidly progressive, neurodegenerative disease that results in the progressive loss of motor neurons that control voluntary muscles. ALS is the most common form of the motor neuron diseases. Oxygen radicals are thought to contribute to the degeneration of motor neurons in ALS, and markers of oxidative stress are upregulated in the tissues of patients.

The reported incidence of ALS varies from 1.26 to 3.98 cases per 100,000 per year, with a prevalence ranging from 4.05 to 7.89 per 100,000 individuals in the European Union (EU), which is equivalent to a total of around 40,000 people. A comparable incidence has been reported in the US. Early symptoms of ALS include stiff muscles and gradual increasing weakness and muscle wasting, which causes progressive degeneration and atrophy of voluntary skeletal muscles ultimately resulting in paralysis. Most patients need assistance with activities of daily living (ADL), with subsequent progression leading to respiratory compromise and eventual respiratory failure, which is a leading cause of death in ALS. The median survival time of ALS patients ranges from of 3 to 5 years. To date four drugs have been approved in the US for the treatment of ALS: the antiglutamatergic agent riluzole, the more recent antioxidant edaravone, tofersen (limited to the use in adults who have a mutation in the superoxide dismutase 1 (*SOD1*) gene) and the drug combination sodium phenylbutyrate and taurursodiol.

Edaravone is the international non-proprietary name (INN) of the chemical compound 5-methyl-2-phenyl-4H-pyrazol-3-one (CAS RN: 89-25-8), which has the following chemical structure:

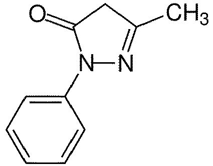

Edaravone, as well as a process for its preparation, was described for the first time in the US patent US460186. Later, the use of edaravone for the treatment of ALS was disclosed in the patent document EP1405637.

Edaravone was approved to Mitsubishi Tanabe by the Food and Drug Administration (FDA) as an intravenous (IV) infusion (60 mg) under the brand name Radicava to treat ALS in 2017. In 2022, edaravone was approved by the FDA as a bioequivalent oral suspension (105 mg, 5 mL) under the brand name Radicava ORS. Both Radicava and Radicava ORS are to be administered following an initial treatment cycle consisting of daily dosing for 14 days followed by a 14-day drug-free period, and subsequent treatment cycles consisting of daily dosing for 10 days out of 14-day periods, followed by 14-day drug-free periods.

The precise mode of action of edaravone in the treatment of ALS is unknown. In cells exposed to oxidative stress, edaravone acts as free radical scavenger and reduces excess oxidative stress and cell death.

Although an attempt was made by Mitsubishi Tanabe to have the edaravone solution for infusion authorized in the European Union, in 2019 they withdrew their marketing authorisation application submitted to the European Medicines Agency (EMA), after the latter requested additional long-term data on survival, and a convincing proposal for post-authorisation studies.

Among the most relevant safety issues identified by the EMA, it was pointed out the need to address the potential risk of neurotoxicity induced by edaravone, based on pre-clinical findings of axonal nerve fibre degeneration, where it was found that edaravone caused reduced vitamin B6 plasma levels. In particular, the EMA noted that edaravone-induced neurotoxicity might have contributed to the imbalanced adverse gait disturbances and respiratory disorders seen in ALS patients treated for 12 months with Radicava (discontinued treatment) compared with the placebo group.

In addition, in the Pharmacology Review of the US FDA on Mitsubishi Tanabe Pharma Corp's edaravone on page 100 (mid of the first full paragraph) it is stated that: 'A mechanism underlying edaravone-induced neurodegeneration was not determined; however, according to the sponsor, vitamin B6 deficits result in a similar pattern of neuropathy. To support such a link, a study in beagle dogs demonstrating a correlation between continuous edaravone infusion and decreasing plasma levels of vitamin B6 was conducted, followed by a second study in which co-administration of vitamin B6 limited the sciatic nerve degeneration induced by continuous edaravone infusion. While these studies suggest that vitamin B6 deficits may play a role in edaravone-induced axonopathy, additional studies are necessary to prove such a mechanism'.

For the time being riluzole is the only approved medication in Europe so far. However, riluzole is indicated for modifying disease progression in ALS by extending life or the time to mechanical ventilation for patients with ALS, but it has no effect on functional aspects of the disease.

Consequently, considering the seriousness of the disease and the limited options for treatment, there remains an urgent and significant unmet medical need for effective treatments of this devastating disease.

### Summary of Invention

According to the withdrawal assessment report of the EMA of Radicava (EMA/CHMP/290284/2019), continuous i.v. infusion of edaravone caused a dose-dependent impaired motor function in dogs from 13 days (300 mg/kg/day) or 18 days of treatment (≥60 mg/kg/day) in the course of the 14 and 28 days repeated-dose toxicity studies. The motor deficits initially started with limited limb usage, tremor, loss of motor coordination and muscle tone in the hindlimbs and then proceeded to the forelimbs one or two days later with increasing severity. The dogs progressively dehydrated, lost body weight and had to be prematurely euthanized between days 22 and 25, if they were unable to stand up. This loss of motor function in dogs was associated with axonal degeneration of peripheral nerves fibres and in the CNS and accounted for the majority of prematurely sacrificed dogs.

In monkeys, motor deficits became eminent in the third and fourth week of the 28 days repeated-dose toxicity study with continuous i.v. infusion of 1000 mg/kg/day edaravone. Similar to dogs, these clinical signs correlated histologically with minimal to severe nerve fibre degenerations with axonal swelling, cellular debris and macrophages/mononuclear cell infiltrates in the central and peripheral nervous system.

Further mechanistic investigations in dogs revealed that the onset of the axonal nerve fibre degenerations was dependent on the continuously infused i.v. dose (day 11 after 300 mg/kg/day and day 15 after 120 mg/kg/day). Of note, nerve fibre degenerations even developed following cessation of edaravone administration in the recovery period, initiated in peripheral nerves and subsequently spread into the spinal cord tissue.

Follow-up mechanistic investigations in dogs indicated a causal relationship of the neurodegenerations with concomitant reductions of vitamin B6. Interestingly, continuous i.v. infusions of 1000 mg/kg/day edaravone reduced the levels of pyridoxal 5'-phosphate (PLP), the active form of vitamin B6, while the combinatorial treatment of edaravone and vitamin B6 in dogs attenuated development and severity of nerve fibre lesions in the peripheral nervous system (PNS).

As indicated in the withdrawal assessment report, edaravone-induce neurotoxicty would be expected in humans and for this reason the EMA still requires for treatments with edaravone: i) early detection of neurotoxicity by monitoring patients for sensory and motor nerve function, and ii) consider vitamin B6 supplementation, which has been associated with attenuation of the development and severity of nerve fibre damage in the PNS.

The alleged neurotoxicity of edaravone might be the cause as well for the 14-day drug-free periods (aka 'holiday period') required for edaravone as authorized by the US FDA.

Contrary to what was expected from the above findings the present inventors surprisingly found that when edaravone is orally administered in a continuous manner, i.e., without drug-free periods, to human patients suffering from an oxidative stress-mediated neurodegenerative disorder, such as Amyotrophic Lateral Sclerosis (ALS), in the morning, at a daily dose ranging from 80 to 120 mg, under certain fasted conditions, a safe and effective treatment is provided, and in particular without the need of vitamin B6 supplementation. Patients that are unable to use the oral route need to be treated by other routes of administration. The gastric administration of edaravore through a feeding tube is considered as equivalent to the oral route of administration.

As illustrated in the examples of the present invention, from the clinical assessment of neuropathy performed during the clinical trial with edaravone, it was found that the percentage of neuropathy in patients was not higher than the percentage found in the normal population. Besides, clinical signs of neuropathy found could be associated to other conditions, such as diabetes or alcoholism.

Furthermore, the results of the Nerve Conduction Study (NCS) also showed that the main criteria for neuropathy: decreased sural nerve SNAP (sensory nerve action potential) amplitudes or decreased sural/radial nerve amplitude ratio (SRAR) were not found in most of the patients, which showed normal values both at baseline and at different times during the study.

Finally, the plasmatic concentration of vitamin B6, monitored at each visit, was not reduced from baseline, which could be considered a sign of neuropathy.

Thus, a first aspect of the invention relates to edaravone or a pharmaceutically acceptable salt thereof for use in the treatment of an oxidative stress-mediated neurodegenerative disorder, wherein the treatment comprises orally or gastrically administering any of them to human subjects in need thereof, once daily in the morning, at a daily dose ranging from 80 to 120 mg, during an uninterrupted period of at least 1 month, wherein human subjects have fasted for at least 2 hours before administration.

This aspect may also be formulated as a method of treatment of an oxidative stress-mediated neurodegenerative disorder, which comprises orally or gastrically administering edaravone or a pharmaceutically acceptable salt thereof, together with one or more pharmaceutically acceptable excipients or carriers, to human subjects in need thereof, once daily in the morning, at a daily dose ranging from 80 to 120 mg, during an uninterrupted period of at least 1 month, wherein human subjects have fasted for at least 2 hours before administration.

It also forms part of the invention the use of edaravone or a pharmaceutically acceptable salt thereof, for the manufacture of a medicament for the treatment of an oxidative stress-mediated neurodegenerative disorder, wherein the treatment comprises orally or gastrically administering any of them to human subjects in need thereof, once daily in the morning, at a daily dose ranging from 80 to 120 mg, during an uninterrupted period of at least 1 month, wherein human subjects have fasted for at least 2 hours before administration.

Additionally, it also forms part of the invention the use of edaravone or a pharmaceutically acceptable salt thereof for use in the treatment of an oxidative stress-mediated neurodegenerative disorder, wherein the treatment comprises orally or gastrically administering any of them to human subjects in need thereof, once daily, at a daily dose ranging from 80 to 120 mg, during an uninterrupted period of at least 1 month, wherein the human subject has fasted for at least 2 hours before administration.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper endpoints of the range.

The term "about" or "around" as used herein refers to a range of values ± 10% of a specified value. For example, the expression "about 10" or "around 10" includes ± 10% of 10, i.e., from 9 to 11.

The term "oxidative stress-mediated neurodegenerative disorder" as used herein refers to a neurodegenerative disorder (i.e., a disorder characterized with progressive deterioration of the structure and/or function of neurons) which is associated to oxidative stress-mediated toxicity, which may involve a number of events including mitochondrial dysfunction, Ca2+ overload, and excitotoxicity. Non-limiting examples of oxidative stress-mediated neurodegenerative disorders that can be treated in the present invention include Amyotrophic Lateral Sclerosis, Alzheimer's disease, multiple sclerosis, cerebral amyloid angiopathy, Parkinson's disease, spinal muscle atrophy, progressive bulbar palsy, primary lateral sclerosis, arthrogryposis multiplex congenita, spinocerebellar degeneration, muscular dystrophy, cerebral infarction, systemic scleroderma, stomatitis, Creutzfeldt-Jakob disease, and Huntington's disease.

The terms "subject in need thereof", "human subject in need thereof" or "patient" are used interchangeably and refer to a human subject that suffers from an oxidative stress-mediated neurodegenerative disorder and may benefit from the treatment provided by the present invention.

The term "daily dose" as used herein refers to the amount of edaravone equivalent, i.e., the amount of edaravone that is contained in a given edaravone salt.

The term "morning" as used herein with respect to the administration time of edaravone or a salt thereof refers to the fact that edaravone or its salt is orally or gastrically administered early in the day, generally after the patient has awakened from overnight sleep, and typically between about 6 a.m. and 11 a.m.

The terms "gastrically administered" or "gastric administration" as used herein refer to administration into the stomach via a tube through the nasal passage such as a nasopharyngeal gastric (NG) tube or a tube in the abdomen such as a percutaneous endoscopic gastrostomy (PEG) tube. The gastric administration via the tube is also referred to as transluminal administration.

The terms "orally administered" or "oral administration" are used in its usual sense and mean that the product is introduced into the organism by the mouth, as opposed, for example, to a mode of parenteral administration.

The term "fasting" as used herein refers to a state where the patient is completely deprived of food but has access to water.

The expression "therapeutically effective amount" as used herein, refers to the amount of edaravone or pharmaceutically acceptable salt thereof which, when administered to a patient, is sufficient to treat, ameliorate or reduce the symptoms of an oxidative stress-mediated neurodegenerative disorder. The specific dose of edaravone or its salts to obtain a therapeutic benefit may vary depending on the particular circumstances of the individual patient including, among others, the type of patient, and the nature and stage of the disease or condition.

The term "pharmaceutically acceptable excipients" refers to components which are appropriate for use in pharmaceutical technology for the preparation of compositions for medical use. Each component should be "acceptable" in the sense of being compatible with the other ingredients of the composition. When used in contact with the tissue or organ of humans and animals should not have excessive toxicity, irritation, allergic response, immunogenicity, or other problems or complications, commensurate with a reasonable benefit/risk ratio.

As mentioned above, the invention provides a novel treatment of human patients suffering from an oxidative stress-mediated neurodegenerative disorders such as Amyotrophic Lateral Sclerosis (ALS) by administering edaravone or a pharmaceutically acceptable salt thereof.

There is no particular limitation on the type of salt of edaravone that can be used, provided that these are pharmaceutically acceptable when they are used for therapeutic purposes. The term "pharmaceutically acceptable salts" embraces non-toxic salts commonly used. The preparation of pharmaceutically acceptable salts of edaravone can be carried out by methods known in the art. For instance, they can be prepared from edaravone by reacting it with a stoichiometric amount of an appropriate pharmaceutically acceptable inorganic or organic acid, in water or in an organic solvent or in a mixture of them. Non-limiting examples of inorganic acids that can be used for preparing edaravone salts include hydrochloric acid, hydrobromic acid or phosphoric acid. Non-limiting examples of organic acids that can be used for preparing edaravone salts include methanesulfonic acid, p-toluenesulfonic acid, acetic acid, oxalic acid, citric acid, and malic acid. Salts of edaravone may also be formed with alkali metals such as sodium or potassium, alkaline earth metals such as magnesium, or with amines such as ammonia, ethanolamine, and 2-amino-2-methyl-1-propanol. Edaravone and its salts may differ in some physical properties, but they are equivalent for the purposes of the present invention.

Edaravone or its salts may be in crystalline form either as free solvation compounds or as solvates (e.g., hydrates). It is intended that both forms are within the scope of the present invention. Methods of solvation are generally known within the art. In general, the solvated forms with pharmaceutically acceptable solvents such as water, ethanol and the like are equivalent to the unsolvated form for the purposes of the invention.

In the treatment of the present invention, edaravone or a salt thereof is orally or gastrically administered to human subjects in need thereof at a daily dose ranging from 80 to 120 mg.

According to one embodiment, optionally in combination with one or more features of the various embodiments described above or below throughout all the description, edaravone or a salt thereof is administered orally.

In another embodiment, optionally in combination with one or more features of the various embodiments described above or below throughout all the description, edaravone or a salt thereof is administered gastrically.

In another embodiment, optionally in combination with one or more features of the various embodiments described above or below throughout all the description, edaravone or a salt thereof is administered at a daily dose of about 80 mg, of about 85 mg, of about 90 mg, of about 95 mg, of about 100 mg, of about 105 mg, of about 110 mg, of about 115 mg, or of about 120 mg.

In another embodiment, optionally in combination with one or more features of the various embodiments described above or below throughout all the description, edaravone or a salt thereof is administered at a daily dose ranging from 90 to 110 mg, more particularly ranging from 100 to 105 mg, and even more particularly at a daily dose of about 100 mg.

Edaravone or a salt thereof are to be administered preferably in the morning, with the condition that the human subject has fasted for at least 2 hours before administration. This means that no food except water has been consumed by the patient at least 2 hours before the administration of edaravone or its salt.

According to one embodiment, optionally in combination with one or more features of the various embodiments described above or below throughout all the description, human subjects have fasted for at least 2 hours, at least 3 hours, at least 4 hours, at least 5 hours, for at least 6 hours, at least 7 hours, at least 8 hours, at least 9 hours, or at least 10 hours.

In another embodiment, optionally in combination with one or more features of the various embodiments described above or below throughout all the description, human subjects have fasted for at least 6 hours, particularly for at least 8 hours before oral administration.

In another embodiment, optionally in combination with one or more features of the various embodiments described above or below throughout all the description, no food except water is consumed by patients in a period of at least 30 min, particularly of at least 1 hour, after the administration of edaravone or a salt thereof.

Unlike previous treatments with edaravone, the treatment of the present invention is a continuous treatment and is carried out during an uninterrupted period of at least 1 month. For the purposes of the invention, the term "uninterrupted period" refers to the fact that during this period there is no pause or drug holiday period in the administration of edaravone or as salt thereof.

According to one embodiment, optionally in combination with one or more features of the various embodiments described above or below throughout all the description, edaravone or a salt thereof is administered during an uninterrupted period of at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months.

In another embodiment, optionally in combination with one or more features of the various embodiments described above or below throughout all the description, edaravone or a salt thereof is administered during an uninterrupted period of at least 3 months, more particularly of at least 6 months, more particularly of at least 12 months, more particularly of at least 18 months and even more particularly, chronically.

In another embodiment, optionally in combination with one or more features of the various embodiments described above or below throughout all the description, the oxidative stress-mediated neurodegenerative disorder is selected from the group consisting of Amyotrophic Lateral Sclerosis, Alzheimer's disease, multiple sclerosis, cerebral amyloid angiopathy, Parkinson's disease, spinal muscle atrophy, progressive bulbar palsy, primary lateral sclerosis, arthrogryposis multiplex congenita, spinocerebellar degeneration, muscular dystrophy, cerebral infarction, systemic scleroderma, stomatitis, Creutzfeldt-Jakob disease, and Huntington's disease. More particularly, the oxidative stress-mediated neurodegenerative disorder is Amyotrophic Lateral Sclerosis or Alzheimer's disease.

In one particular embodiment, optionally in combination with one or more features of the various embodiments described above or below throughout all the description, the oxidative stress-mediated neurodegenerative disorder is Amyotrophic Lateral Sclerosis (ALS).

In another particular embodiment, optionally in combination with one or more features of the various embodiments described above or below throughout all the description, edaravone or a salt thereof is administered concurrently, separately or sequentially in combination with riluzole.

ALS is primarily a clinical diagnosis and may be difficult to diagnose with certainty in early stages. In 1994 the World Federation of Neurology published the EI Escorial criteria for diagnosing ALS. The EI Escorial ALS diagnostic criteria were revised in 1998 and includes 4 diagnostic categories: Definite ALS, Probable ALS, laboratory results supported-Probable ALS, and Possible ALS. "Definite ALS" refers to the presence of upper motor neuron and lower motor neuron signs in three anatomical regions. "Probable ALS" refers to the presence of upper motor neuron and lower motor neuron signs in at least two regions with upper motor neuron sign rostral to lower motor neuron signs. "Laboratory results supported-Probable ALS" refers to the presence of upper motor neuron and lower motor neuron signs in one region with evidence by electromyography (EMG) of lower motor neuron involvement in another region. "Possible ALS" refers to the presence of upper motor neuron and lower motor neuron signs in one region or upper motor neuron signs in two or three regions, such as monomelic ALS, progressive bulbar palsy, and primary lateral sclerosis.

On the other hand, motor impairment and functional deterioration in people with ALS can be measured by a severity score known as ALS Functional Rating Scale. The Revised Amyotrophic Lateral Sclerosis Functional Rating Scale (ALSFRS-R) has been widely applied in both clinical practice and ALS research. The scale encompasses 12 prompts-referred to as items-grouped into four domains to assess bulbar symptoms, limb and trunk functionality, respiratory symptoms, and the need for percutaneous endoscopic gastrostomy, non-invasive ventilation, or tracheostomy with invasive ventilation. To ordinally scale the loss of functionality, anchor points grade functionality from 0 to 4 for each item. Score 4 designates unrestricted functionality. Score 3 is related to a mild impairment and reflects a condition that does not yet require compensatory help. Score 2 is characterized by intermittent use of compensatory measures. Score 1 is given if assistive procedures or devices are needed in all instances, and independence is severely reduced but not entirely lost. Finally, score 0 designates a complete loss of functionality.

According to one embodiment, optionally in combination with one or more features of the various embodiments described above or below throughout all the description, edaravone or a salt thereof is administered to human subjects having a progression of the disease such that the total score variation of the Revised Amyotrophic Lateral Sclerosis Functional Rating Scale (ALSFRS-R) is lower than 1 point per month.

In another embodiment, optionally in combination with one or more features of the various embodiments described above or below throughout all the description, edaravone or a salt thereof is initially administered to human subjects which have been diagnosed with Amyotrophic Lateral Sclerosis with an onset of symptoms of less than 24 months, more particularly of more than 1 month.

In another embodiment, optionally in combination with one or more features of the various embodiments described above or below throughout all the description, edaravone or a salt thereof is administered to human subjects which have been diagnosed with Definite ALS or Probable ALS based on EI Escorial revised criteria.

The use of edaravone or a pharmaceutically acceptable salt thereof for use in the treatment of an oxidative stress-mediated neurodegenerative disorder, wherein the treatment comprises orally or gastrically administering any of them to human subjects in need thereof, once daily, at a daily dose ranging from 80 to 120 mg, during an uninterrupted period of at least 1 month, wherein the human subjects have fasted for at least 2 hours before administration, is surprisingly associated with reduced signs of neurotoxicity, such as the decrease in the values of both the sensory nerve action potential amplitude (SNAP Amp) and the sural-radial Amp ratio as shown in the examples.

Thus, in another embodiment, optionally in combination with one or more features of the various embodiments described above or below throughout all the description, less than 15 % of the human subject show a shift from baseline from normal to low values of sensory nerve action potential amplitude (SNAP Amp) for the sural nerve, preferably 10 % or less, more preferably 7 % or less; and less than 20 % of the human subject show a shift from baseline from normal to low values in the sural-radial Amp ratio during the treatment, preferably 15 % or less, more preferably 10 % or less. Additionally, these human subjects could be treated once daily at any time of the day.

Normal values for the sensory nerve action potential amplitude (SNAP Amp) for the sural nerve are values higher than 8 µV for subjects with less than 40 years of age, higher than 5 µV for subjects with 40-60 years of age, and higher than 2 µV for subjects with more than 60 years of age.

Normal values for the sural-radial Amp ratio (SRAR) are values higher than 0.45 for subjects with 18-39 years, higher than 0.32 for subjects with 40-59 years and higher than 0.19 for subjects with 60 or more years of age.

As mentioned above, contrary to what was expected the continuous administration of edaravone under the conditions as defined herein is safe and does not cause neurotoxicity. Therefore, there is no need for vitamin B6 supplementation during the treatment with edaravone or a salt thereof. Thus, in one embodiment, optionally in combination with one or more features of the various embodiments described above or below throughout all the description, edaravone or a salt thereof is administered in the absence of B6 supplementation.

In another embodiment, optionally in combination with one or more features of the various embodiments described above or below throughout all the description, human subjects are treated without the need of vitamin B6 supplementation, particularly without vitamin B6 supplementation. Additionally, these human subjects could be treated once daily at any time of the day.

In another embodiment, optionally in combination with one or more features of the various embodiments described above or below throughout all the description, the plasma concentration of pyridoxal 5'-phosphate in human subjects during the treatment equals or exceeds 12 ng/L, preferably exceeds 30 ng/L. Additionally, these human subjects could be treated once daily at any time of the day.

In another embodiment, optionally in combination with one or more features of the various embodiments described above or below throughout all the description, the plasma concentration of pyridoxal 5'-phosphate in the human subject at the end of the uninterrupted treatment period equals or exceeds 12 ng/L, preferably exceeds 30 ng/L.

Typically, edaravone or a salt thereof is administered in the form of a pharmaceutical composition. Thus, according to one embodiment, optionally in combination with one or more features of the various embodiments described above or below throughout all the description, edaravone or a salt thereof is administered in the form of a pharmaceutical composition which comprises a therapeutically effective amount of edaravone or a pharmaceutically acceptable salt thereof, together with one or more pharmaceutically acceptable excipients.

For the purposes of the invention, there is no particular limitation on the type of formulation that can be used. Thus, liquid and solid formulations may be used.

According to one embodiment, optionally in combination with one or more features of the various embodiments described above or below throughout all the description, the pharmaceutical composition is a liquid formulation, more particularly selected from a solution and a suspension.

In one embodiment, optionally in combination with one or more features of the various embodiments described above or below throughout all the description, the pharmaceutical composition is a solution, more particularly an aqueous solution.

In one more particular embodiment, optionally in combination with one or more features of the various embodiments described above or below throughout all the description, the pharmaceutical composition is a solution having a pH in the range from 6.0 to 9.0, particularly in the range from 6.5 to 8.8, and more particularly in the range from 6.8 to 8.5.

In another more particular embodiment, optionally in combination with one or more features of the various embodiments described above or below throughout all the description, the concentration of edaravone or a pharmaceutically acceptable salt thereof in the solution ranges from 0.5 to 3 mg/mL, more particularly from 0.8 to 2 mg/mL, and even more particularly of 1 to 2 mg/mL.

In another more particular embodiment, optionally in combination with one or more features of the various embodiments described above or below throughout all the description, the solution is administered to the subject in need thereof in an amount ranging from 30 to 200 mL, particularly from 50 to 200 mL, more particularly from 50 to 150 mL, and even more particularly in an amount of about 50 mL, about 75 mL, about 100 mL, or about 150 mL.

In another more particular embodiment, optionally in combination with one or more features of the various embodiments described above or below throughout all the description, the solution comprises a filler, a pH adjusting agent, and a surfactant.

Non-limiting examples of fillers that can be used that can be used in the present invention include mannitol, sorbitol, xylitol, maltitol, erythritol, sucrose, trehalose, lactose, maltose, glucose, glycerin, pregelatinized starch, microcrystalline cellulose, cellulose, dibasic calcium phosphate, or combinations thereof.

Non-limiting examples of pH adjusting agents that can be used in the present invention include hydrochloric acid, phosphoric acid, acetic acid, citric acid, tartaric acid, a lactate salt, a citrate salt, an acetate salt, a formate salt, an oxalate salt, a phosphate salt, a sulfate salt, sodium hydroxide, potassium hydroxide, calcium carbonate, magnesium oxide, magnesium hydroxide, or combinations thereof.

Non-limiting examples of surfactants that can be used in the present invention include sodium lauryl sulfate, docusate sodium, sorbitan fatty acid esters, polysorbates, polyoxyethylene alkyl ethers, poloxamers, medium-chain triglycerides, polyoxylglycerides, polyoxyethylene castor oil derivatives.

According to one embodiment, optionally in combination with one or more features of the various embodiments described above or below throughout all the description, the solution comprises:
a) from 0.05 to 3% by weight of edaravone or a pharmaceutically acceptable salt thereof, particularly edaravone;
b) from 0.1 to 6% by weight of a filler, particularly mannitol;
c) from 0.1 to 2% by weight of a pH adjusting agent, particularly disodium hydrogen phosphate;
d) from 0.001 to 0.5% by weight of a surfactant, particularly sodium lauryl sulfate;
wherein the weight percentages are expressed with respect to the total composition weight, and with the proviso that the sum of all the components of the composition is 100%.

The edaravone solution to be administered may be prepared shortly before its administration. Thus, in one embodiment, optionally in combination with one or more features of the various embodiments described above or below throughout all the description, the solution is prepared just before its administration by mixing a solid particulate formulation comprising edaravone or a pharmaceutically acceptable salt thereof together with one or more pharmaceutically acceptable excipients, with an aqueous liquid, particularly one comprising water, more particularly water.

In one particular embodiment, optionally and in combination with one or more features of the various embodiments described above or below throughout all the description, the solid particulate formulation comprises granules of edaravone or a pharmaceutically acceptable salt thereof.

In another particular embodiment, optionally and in combination with one or more features of the various embodiments described above or below throughout all the description, the solid particulate formulation further comprises one or more pharmaceutically acceptable excipients, more particularly a filler, a pH adjusting agent, and a surfactant.

In another more particular embodiment, optionally in combination with one or more features of the various embodiments described above or below throughout all the description, the solid particulate formulation comprises granules of edaravone in an amount of about 1.5%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%, by weigh with respect to the total weight of the solid particulate formulation.

In another more particular embodiment, optionally in combination with one or more features of the various embodiments described above or below throughout all the description, the solid particulate formulation comprises granules of edaravone in an amount ranging from 1.5 to 20%, more particularly from 5 to 15%, by weigh with respect to the total weight of the solid particulate formulation.

In another more particular embodiment, optionally in combination with one or more features of the various embodiments described above or below throughout all the description, the solid particulate formulation comprises a filler, more particularly mannitol, in an amount ranging from 30 to 90 %, more particularly from 45 to 70 %, by weigh with respect to the total weight of the solid particulate formulation.

In another more particular embodiment, optionally in combination with one or more features of the various embodiments described above or below throughout all the description, the solid particulate formulation comprises a pH adjusting agent, more particularly disodium hydrogen phosphate, in an amount ranging from 20 to 50 %, more particularly from 25 to 45 %, by weigh with respect to the total weight of the solid particulate formulation.

In another more particular embodiment, optionally in combination with one or more features of the various embodiments described above or below throughout all the description, the solid particulate formulation comprises a surfactant, more particularly sodium lauryl sulfate, in an amount ranging from 0.02 to 4 %, more particularly from 0.2 to 2 %, by weigh with respect to the total weight of the solid particulate formulation.

In another embodiment, optionally in combination with one or more features of the various embodiments described above or below throughout all the description, the pharmaceutical composition is a suspension, more particularly a suspension having a pH in the range from 2.5 to 6.0.

In another embodiment, optionally in combination with one or more features of the various embodiments described above or below throughout all the description, edaravone or a pharmaceutically acceptable salt thereof is present in the suspension in the form of particles having a Dv50 particle size from 10 to 100 µm, and a D90 particle size from 90 to 300 µm.

For the purposes of the invention, Dv50, also referred to as median particle size by volume, is the maximum particle diameter below which 50% of the sample volume exists. Dv90 is the maximum particle diameter below which 90% of the sample volume exists. Particle size may be measured by methods well-known in the art such as for example laser diffraction.

In another embodiment, optionally in combination with one or more features of the various embodiments described above or below throughout all the description, the suspension has a viscosity from 50 to 1750 mPa·s.

In another embodiment, optionally in combination with one or more features of the various embodiments described above or below throughout all the description, the suspension has a density from 1 to 1.5 g/mL.

In another embodiment, optionally in combination with one or more features of the various embodiments described above or below throughout all the description, the suspension comprises a filler, a dispersant agent, a thickening agent, an antioxidant, an antifoaming agent, and a pH adjusting agent.

Non-limiting examples of dispersant agents that can be used in the present invention include polyvinyl alcohol, methylcellulose, hypromellose, sucrose fatty acid ester and polysorbate, or combinations thereof.

Non-limiting examples of thickening agents that can be used in the present invention include xanthan gum, tragacanth powder, or combinations thereof.

Non-limiting examples of antioxidants that can be used in the present invention include a sulfite, a bisulfite, a pyrosulfite, cysteine, or combinations thereof.

Non-limiting examples of pH antifoaming agents that can be used in the present invention include simethicone, a fatty acid ester, a polysorbate, ethanol, or combinations thereof.

According to one embodiment, optionally in combination with one or more features of the various embodiments described above or below throughout all the description, the suspension comprises:
a) from 0.5 to 6% by weight of edaravone or a pharmaceutically acceptable salt thereof, particularly edaravone;
b) from 10 to 60% by weight of a filler, particularly sorbitol;
c) from 0.001 to 1.0% by weight of a dispersant, particularly polyvinyl alcohol;
d) from 0.1 to 1.2% by weight of a thickening agent, particularly xanthan gum;
e) from 0.01 to 0.5% by weight of an antioxidant, particularly L-cysteine hydrochloride hydrate and sodium bisulfite;
f) from 0.01 to 0.05% by weight of an antifoaming agent, particularly simethicone;
g) a pH adjusting agent, particularly phosphoric acid and sodium hydroxide, in an amount to adjust the pH of the suspension to a pH from 2.5 to 6.0;
wherein the weight percentages are expressed with respect to the total composition volume, and with the proviso that the sum of all the components of the composition is 100%.

In another embodiment, optionally in combination with one or more features of the various embodiments described above or below throughout all the description, the pharmaceutical composition is a solid oral formulation.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

### A. Preparation of the pharmaceutical composition

### 1. Quantitative composition

Edaravone granules for oral solution (in sachet) is a white to off-white granular powder, containing 100 mg of edaravone per sachet. The content of the sachet is to be reconstituted in approximately 50-100 mL of water prior to administration.

The theoretical quantitative composition of the prepared edaravone granules for oral solution in sachet is summarized in Table 1.

**Table 1. Theoretical quantitative composition of edaravone granules for oral solution per sachet, 100 mg.**

| **Component** | **Reference to standard** | **Function** | **Amount (mg)** | **(w/w) %** |
|---|---|---|---|---|
| Edaravone | In-house | Active pharmaceutical ingredient | 100.0 | 9.17 |
| Mannitol | Ph.Eur./USP-NF | Filler | 621.8 | 57.00 |
| Disodium hydrogen phosphate dihydrate¹ (OP1) | Ph.Eur./USP-NF | pH adjusting agent | 363.6 | 33.33 |
| Sodium Laurilsulfate² (SLS) | Ph.Eur./USP-NF | Surfactant | 5.5 | 0.50 |
| Total | | | 1090.9 | 100.00 |

| | | | | |
|---|---|---|---|---|
| ¹ Ph.Eur.: Disodium Phosphate Dihydrate; USP: Dibasic Sodium Phosphate. It will be referred to as OP1 throughout the document. ² Ph.Eur.: Sodium Laurilsulfate; USP-NF: Sodium Lauryl Sulfate. It will be referred to as SLS throughout the document. | | | | |

The product is prepared by filling bulk granulate obtained by following the batch formula and manufacturing process detailed below into sachets. Before filling, an assay of the content of edaravone in the bulk is carried out and the amount of bulk granulate to be placed in each sachet is adjusted to ensure that each sachet contains 100 mg of edaravone.

### 2. Batch Formula of bulk granulates

Different batch sizes were prepared:
- Batch size 40 kg, equivalent to approx. 36,667 sachets.
- Batch size 60 kg, equivalent to approx. 55,000 sachets.
- Batch size 80 kg, equivalent to approx. 73,334 sachets.

The composition of the prepared batches is shown in Table 2.

**Table 2. Composition of 40, 60 and 80 kg batches of edaravone granules for oral solution in sachet, 100 mg per sachet**

| **Component** | | **40 kg batch formula Amount (kg)/batch** | **60 kg batch formula Amount (kg)/batch** | **80 kg batch formula Amount (kg)/batch** |
|---|---|---|---|---|
| Edaravone | | 3.667 | 5.500 | 7.333 |
| Mannitol | | 22.800 | 34.199 | 45.599 |
| Disodium hydrogen phosphate dihydrate¹ (OP1) | | 13.332 | 19.998 | 26.664 |
| Sodium Laurilsulfate² (SLS) | | 0.202 | 0.303 | 0.403 |
| TOTAL | 100.00% | 40.000 | 60.000 | 80.000 |

| | | | | |
|---|---|---|---|---|
| ^{F1} Ph.Eur.: Disodium Phosphate Dihydrate; USP: Dibasic Sodium Phosphate. ² Ph.Eur.: Sodium Laurilsulfate; USP-NF: Sodium Lauryl Sulfate. | | | | |

### 3. Manufacturing process

The above compositions were prepared following the process shown below:

**Table 3**

| **Process step** | **Description** |
|---|---|
| 1 | Granulation |
| | Weigh and sieve the raw materials through a mesh sieve appropriate amounts of edaravone, mannitol, SLS, milled OP1. |
| | Place previous raw materials sieved in the high shear mixer and blend all the components until homogeneity. |
| | Granulate the powder blend in the high shear mixer using the appropriate amount of purified water (Approx 15-20%). |
| | Dry the granules in fluid bed dryer until LoD (loss on drying) value after drying is below specification. LoD NMT 7.4%. |
| | Sieve the granules through a small mesh sieve. |
| 2 | Blend |
| | Blend the sieved granules in a blender equipment until homogeneity. |
| 3 | Sachet filling |
| | Fill the appropriate amount of powder into a sachet (in case of assay <100.0 %, the fill weight is adjusted). |
| | Seal the sachets. |

Step 1 can be repeated when the size of equipment warrants manufacturing of identical fractions of the same batch, subsequently brought together to form a final homogenous batch.

### B. Multicenter, randomized, double-blind, placebo-controlled study to investigate the efficacy and safety of edaravone granules for oral solution in patients with Amyotrophic Lateral Sclerosis

A clinical study was started to investigate the efficacy and safety of 100 mg of edaravone granules for oral solution (composition shown in table 1 above) in 300 patients with Amyotrophic Lateral Sclerosis (ALS) following treatment for 48 or 72 weeks. The first 100 subjects enrolled will have either 72 weeks of follow-up or have reached an intercurrent event, and the rest (about two thirds of enrolled patients) will be followed up to 48 weeks (or have reached an intercurrent event).

This study is currently ongoing, and results are provided at a given point in time of the patients assessed at that time. As patients are included in the study one by one, data at week 72 (end of the study) is only available for the first randomized patients.

The eligibility criteria were as follows:
1. Age: 18 - 80 years;
2. Diagnosis of ALS;
3. Disease onset within the last 24 months;
4. Slow Vital Capacity (SVC) 70% or higher at the screening visit;
5. Change in ALSFRS-R (Amyotrophic Lateral Sclerosis -ALS- Functional Rating Scale in its revised version) between 0.35 points and 1.5 points per month (both inclusive) in the period from onset of first symptoms to the screening visit;
6. In case patients were taking riluzole, patients must be on stable doses ≥30 days prior to the baseline visit (and the dose must be maintained during the entire trial);
7. Female participants not pregnant or breast-feeding during the entire trial.

### Arms and Interventions:

During the treatment, the participants are randomly assigned to receive a daily dose of 100 mg of either edaravone (composition shown in table 1 above) or placebo at a ratio of 2:1 (i.e., two thirds of the patients receive the edaravone formulation and one third receives placebo) for 72 weeks. The study medication, both the placebo and edaravone, is available in single-dose sachets, which must be dissolved in water and taken orally once daily in the morning after a lapse of at least 2 hours from the last meal consumption.

During the study patients were asked to attend several medical visits in person:
- To review and check the screening criteria (inclusion and exclusion)
- To confirm their participation in the study (baseline visit)
- At week 4
- At week 12
- Every 12 weeks thereafter

Within the framework of the above clinical trial the results of a Nerve Conduction Study (NCS), simultaneously with the assessment on vitamin B6, clinical signs, and ALSFRS-R (Amyotrophic Lateral Sclerosis -ALS- Functional Rating Scale in its revised version) including questions related to leg functioning (8. Walking and 9. Climbing stairs) were assessed.

Several authors and regulatory agencies (see for instance EMA/CHMP/290284/2019, page 134) consider that monitoring of the levels of pyridoxal 5'-phosphate (PLP), the active form of vitamin B6, and testing of motor or sensory nerve conduction, such as Nerve Conduction Studies (NCS) described below, should be carried out in order to allow for early detection of neurotoxicity. In addition to this, clinical assessment of neuropathy of patients was carried out by neurologist.

Previous animal toxicology studies revealed neurologic adverse events (e.g. abnormal gait, decreased activity, prostration, reduced function of the hind limbs) with prolonged exposure to edaravone at relatively low safety margins compared to the anticipated human exposure levels. Analysis of the non-clinical data indicated that the neurotoxic effects seen after edaravone administration resulted from demyelination (primary effect) followed by axonal degeneration (secondary effect) and these events were related to reduced levels of pyridoxal 5'-phosphate (PLP), the active form of vitamin B6, in plasma. Therefore, potential neurotoxicity by NCS assessment and by PLP levels during the conduct of the current study was monitored.

### 1. Clinical assessment of neuropathy

According to the assessment of a centralized evaluation team of neurologists, out of 313 randomized patients taking edaravone or placebo at 2:1 ratio, only 7 of them (2.2 %) showed clinical signs of neuropathy after being treated for periods ranging from 6 months to one year and 7 months. This percentage of neuropathy was not higher than the percentage found in the normal population of about 8 %. Moreover, the percentage of neuropathy found in patients suffering from other chronic diseases such as diabetes, HIV infection or alcoholism is higher than 20 %. When the blinding of the study is broken, the specific percentages of neuropathy could be linked to the edaravone and placebo arms. In any case, since two-thirds of the patients were taking edaravone, this result proves that unexpectedly edaravone treatment is not associated with neuropathy and is less neurotoxic than expected.

In addition, clinical signs of neuropathy found could be associated to other conditions, such as diabetes or alcoholism.

### 2. Nerve Conduction Study (NCS)

A centralized evaluation was performed by neurophysiologists to avoid any bias between sites and to guarantee the safety of patients. The NCS was initially performed for the first 60 enrolled patients at screening, and on weeks 4, 12, and 24. This data was reviewed by an independent Data Safety Monitoring Board (DSMB) and, as no neurological concerns were observed, due to the low incidence of potential neuropathies diagnosed in this population, their recommendation was that only an additional NCS was to be performed at week 48 for this first 60 patients, and for the rest of participants, included after that decision was taken, a NCS was only necessary at screening and at week 48 of treatment.

Baseline data including NCS results on the first 196 enrolled patients at screening are available. The main aim of the intensive monitoring of patients with the NCS is to identify subclinical damage of sensory function before the potential development of clinically significant neuropathy. The relevant expected clinical presentation of neuropathy is predominantly sensory and includes relative symmetric paraesthesia and/or numbness mainly in feet.

NCS main criteria for neuropathy are decreased sural nerve SNAP (sensory nerve action potential) amplitudes or decreased sural/radial nerve amplitude ratio (SRAR), although all data is assessed globally.

Thus, when a patient initially shows 'normal' values of one of these parameters and during the treatment these values become 'low' this might be a sign of the possible development of neuropathy. The fact that most of the patients show no change in these values, i.e., that they start with 'normal' and remain 'normal' is a sign of lack of neurotoxicity of the treatment. The appearance of neuropathy may also occur at the normal population for other causes such as diabetes or alcoholism, totally unrelated to any treatment.

### Protocol description

The NCS protocol evaluates the sensory nerve action potential (SNAP) amplitudes using standard ring-electrodes and predefined sites for both stimulus (antidromic) and recording sites, including standardized distances that also apply for the inter-electrode distances. Hence, routine signal averaging for the recorded SNAP's is performed after supramaximal stimulation. These measures eliminate any unwanted variation due to instrumentational differences.

NCS data is evaluated independently at a single specialized unit taking into account reference values provided by participating sites and using age-adjusted reference values. Recordings can be performed on a standard type of electromyograph (EMG) equipment (e.g., Natus Nicolet EDX, Nicolet Viking, Dantec Keypoint, TECA Synergy, Cadwell).

The following steps were followed to perform the NCS assessment:
- Recording and documentation of temperature at start of the assessment at hand and foot respectively. Limb temperature should be ≥ 31°C, otherwise warming may be considered or alternatively accepted correction factor for decreased temperature could be done.
- Unilateral recording of sensory responses of the following nerves (hand, followed by lower limb) was carried out:
   ∘ Median nerve (hand, dig II). The distance from the stimulus and recording electrode should be recorded.
   ∘ Ulnar nerve (hand, dig V). The distance from the stimulus and recording electrode should be recorded.
   ∘ Radial nerve (back of the hand). A standardized distance stimulus-recording electrode of 12 cm should be used.
   ∘ Sural nerve (leg, lateral malleolus). A standardized distance stimulus-recording electrode of 12 cm should be used.
- Recording of sensory responses were carried out using standardized sides (left or right) for stimulation (assessments were performed on one side only, the same side for each subject was used throughout the study). Electrodes were placed at an interelectrode distance of 2 cm. The 12 cm stimulus-recording distance only applies for radial and sural nerves, which should still be feasible for those nerves, and does not apply to median/ulnar nerves (in the latter larger distances are common). For each nerve, sensory nerve action potential (SNAP) amplitudes were evaluated. Antidromic stimulus were provided to the subjects. Required quality of the NCS included: optimization of the recordings, ensuring flat base line without a slope, omitting stimulus artifacts and background noise, so that markers could be placed appropriately, and true SNAP amplitude values could be obtained accurately.

### NCS Ranges of normality

The NCS Ranges of normality considered for this clinical study for SNAP Amp (sensory nerve action potential amplitude) and SCV (sensory conduction velocity) are shown below:

**Table 4**

| Nerve | SNAP Amp / SCV | <40 years | <60 years | 40-60 years | >60 years |
|---|---|---|---|---|---|
| Median nerve | SNAP Amp (µV) | | >11 | | >5 |
| | SCV (m/s) | 30-70 | | | |
| Ulnar nerve | SNAP Amp (µV) | >11 | | >7 | >5 |
| | SCV (m/s) | 30-70 | | | |
| Radial nerve | SNAP Amp (µV) | >11 | | >7 | >5 |
| | SCV (m/s) | 30-70 | | | |
| Sural nerve | SNAP Amp (µV) | >8 | | >5 | >2 |
| | SCV (m/s) | 30-70 | | | |

Normal values for the sensory nerve action potential amplitude (SNAP Amp) for the sural nerve are values higher than 8 µV for subjects with less than 40 years of age, higher than 5 µV for subjects with 40-60 years of age, and higher than 2 µV for subjects with more than 60 years of age.

Normal values for the sural-radial Amp ratio (SRAR) are values higher than 0.45 for subjects with 18-39 years, higher than 0.32 for subjects with 40-59 years and higher than 0.19 for subjects with 60 or more years of age.

Other normal values for this clinical study are shown below:
Ulnar, Radial, Median, Sural Nerve: SNAP Variability is < or = than 25%.
SCV value for the Median, Ulnar, Radial and Sural nerve should be between 30 - 70 m/s. Hand Temperature expected range (30 - 37C).
Foot Temperature is outside expected range (30 - 37C).

### Sural nerve - SNAP Amp

**Table 5. Population: Safety (SAF) (N = 196)**

| **Visit** | **Shift from Baseline** | **Overall (N=196) n (%)** |
|---|---|---|
| Visit 2 (Week 4) | N | 167 |
| Visit 2 (Week 4) - n (%) | Low to Low | 3 (1.8) |
| Visit 2 (Week 4) - n (%) | Low to Normal | 0 (0.0) |
| Visit 2 (Week 4) - n (%) | Normal to Low | 2 (1.2) |
| Visit 2 (Week 4) - n (%) | Normal to Normal | 162 (97.0) |
| Visit 3 (Week 12) | N | 115 |
| Visit 3 (Week 12) - n (%) | Low to Low | 2 (1.7) |
| Visit 3 (Week 12) - n (%) | Low to Normal | 0 (0.0) |
| Visit 3 (Week 12) - n (%) | Normal to Low | 3 (2.6) |
| Visit 3 (Week 12) - n (%) | Normal to Normal | 110 (95.7) |
| Visit 4 (Week 24) | N | 60 |
| Visit 4 (Week 24) - n (%) | Low to Low | 0 (0.0) |
| Visit 4 (Week 24) - n (%) | Low to Normal | 0 (0.0) |
| Visit 4 (Week 24) - n (%) | Normal to Low | 3 (5.0) |
| Visit 4 (Week 24) - n (%) | Normal to Normal | 57 (95.0) |

| | | |
|---|---|---|
| *Percentages are based on the number of subjects in the population of each visit (n).* | | |

### Sural-Radial Amp Ratio (SRAR)

**Table 6. Population: Safety (SAF) (N = 196)**

| **Visit** | **Shift from Baseline** | **Overall (N=196) n (%)** |
|---|---|---|
| Visit 2 (Week 4) | N | 165 |
| Visit 2 (Week 4) - n (%) | Low to Low | 9 (5.5) |
| Visit 2 (Week 4) - n (%) | Low to Normal | 8 (4.8) |
| Visit 2 (Week 4) - n (%) | Normal to Low | 6 (3.6) |
| Visit 2 (Week 4) - n (%) | Normal to Normal | 142 (86.1) |
| Visit 3 (Week 12) | N | 112 |
| Visit 3 (Week 12) - n (%) | Low to Low | 5 (4.5) |
| Visit 3 (Week 12) - n (%) | Low to Normal | 4 (3.6) |
| Visit 3 (Week 12) - n (%) | Normal to Low | 10 (8.9) |
| Visit 3 (Week 12) - n (%) | Normal to Normal | 93 (83.0) |
| Visit 4 (Week 24) | N | 59 |
| Visit 4 (Week 24) - n (%) | Low to Low | 4 (6.8) |
| Visit 4 (Week 24) - n (%) | Low to Normal | 1 (1.7) |
| Visit 4 (Week 24) - n (%) | Normal to Low | 4 (6.8) |
| Visit 4 (Week 24) - n (%) | Normal to Normal | 50 (84.7) |

| | | |
|---|---|---|
| *Percentages are based on the number of subjects in the population of each visit (n).* | | |

### 3. Vitamin B6 monitoring

Plasmatic concentration of vitamin B6 was monitored at each visit. The normal range of plasma concentration of vitamin B6 in humans (measured as pyridoxal 5'-phosphate) is 12-128 ng/L, values below this range are considered 'low' and values above, 'high'. In the inclusion visit at time zero patients were classified as 'normal', 'low' or 'high' and the change of this classification was assessed at each visit.

Some authors consider that low plasmatic levels of vitamin B6 are a possible sign of neuropathy. In this sense, the European Medicine Agency requires to monitor vitamin B6 plasma levels in clinical trials for drugs showing potential neurotoxicity.

The fact that most of the patients show no change in the classification as 'normal', 'low', or 'high' during the study is a sign of low neurotoxicity of the product. This is the case for patient showing no shift from baseline, for instance 'Normal to Normal' patients.

In addition, the Summary table below shows that patients do not show a decrease in the plasmatic concentrations of vitamin B6 (measured as pyridoxal 5'-phosphate) during the study as the mean value does not decrease over time.

Therefore, there is no sign of neurotoxicity associated to a decrease of vitamin B6.

**Table 7**

| **Visit** | **Shift from Baseline at Visit 1** | **Overall N=196 #** | **%** |
|---|---|---|---|
| **Visit 2 (Week 4)** | Total # of patients visited | 157 | |
| | Low to Low | 0 | 0.0 |
| | Low to Normal | 3 | 1.9 |
| | Low to High | 1 | 0.6 |
| | Normal to Low | 0 | 0.0 |
| | Normal to Normal | 127 | 80.9 |
| | Normal to High | 4 | 2.5 |
| | High to Low | 0 | 0.0 |
| | High to Normal | 7 | 4.5 |
| | High to High | 15 | 9.6 |
| **Visit 3 (Week 12)** | Total # of patients visited | 109 | |
| | Low to Low | 0 | 0.0 |
| | Low to Normal | 1 | 0.9 |
| | Low to High | 1 | 0.9 |
| | Normal to Low | 0 | 0.0 |
| | Normal to Normal | 86 | 78.9 |
| | Normal to High | 5 | 4.6 |
| | High to Low | 0 | 0.0 |
| | High to Normal | 8 | 7.3 |
| | High to High | 8 | 7.3 |
| **Visit 4 (Week 24)** | Total # of patients visited | 59 | |
| | Low to Low | 0 | 0.0 |
| | Low to Normal | 0 | 0.0 |
| | Low to High | 0 | 0.0 |
| | Normal to Low | 0 | 0.0 |
| | Normal to Normal | 43 | 72.9 |
| | Normal to High | 4 | 6.8 |
| | High to Low | 0 | 0.0 |
| | High to Normal | 6 | 10.2 |
| | High to High | 6 | 10.2 |
| **Visit 5 (Week 36)** | Total # of patients visited | 12 | |
| | Low to Low | 0 | 0.0 |
| | Low to Normal | 0 | 0.0 |
| | Low to High | 0 | 0.0 |
| | Normal to Low | 0 | 0.0 |
| | Normal to Normal | 5 | 41.7 |
| | Normal to High | 0 | 0.0 |
| | High to Low | 0 | 0.0 |
| | High to Normal | 4 | 33.3 |
| | High to High | 3 | 25.0 |
| **Visit 8 (Week 72/End of Trial)** | Total # of patients visited | 2 | |
| | Low to Low | 0 | 0.0 |
| | Low to Normal | 0 | 0.0 |
| | Low to High | 0 | 0.0 |
| | Normal to Low | 0 | 0.0 |
| | Normal to Normal | 2 | 100.0 |
| | Normal to High | 0 | 0.0 |
| | High to Low | 0 | 0.0 |
| | High to Normal | 0 | 0.0 |
| | High to High | 0 | 0.0 |

| | | | |
|---|---|---|---|
| *Percentages are based on the number of subjects visited at each visit.* | | | |

**Table 8. Summary Table**

| | | **Overall (N=196)** | | |
|---|---|---|---|---|
| **Visit** | **Statistic** | **Observed value** | **SD** | **Change from baseline** |
| Screening | n | 182 | | |
| | Mean | 74.90 | 100.5 | |
| | Median | 41.50 | | |
| Visit 1 (Baseline) | N | 163 | | |
| | Mean | 76.6 | 97.3 | |
| | Median | 41.0 | | |
| Visit 2 (Week 4) | N | 159 | | -4 |
| | Mean | 77.10 | 94.8 | 0.5 |
| | Median | 43.0 | | 2.0 |
| Visit 3 (Week 12) | n | 109 | | -163 |
| | Mean | 69.80 | 79.2 | -6.8 |
| | Median | 43.0 | | 2.0 |
| Visit 4 (Week 24) | n | 59 | | -104 |
| | Mean | 75.9 | 84.3 | -0.7 |
| | Median | 45.0 | | 4.0 |
| Visit 5 (Week 36) | n | 12 | | -151 |
| | Mean | 95.8 | 97.9 | 19.2 |
| | Median | 74.5 | | 33.5 |

| | | | | |
|---|---|---|---|---|
| n: Total # of patients visited; SD: standard deviation. | | | | |

### Citation List

- US460186
- EP1405637
- EMA/CHMP/290284/2019:EMA/CHMP/290284/2019 Committee for Medicinal Products for Human Use (CHMP); Withdrawal assessment report: Radicava; 24 May 2019. Available on: https://www.ema.europa.eu/en/documents/withdrawal-report/withdrawal-assessment-report-radicava en.pdf
- Pharmacology Review of the US FDA on Mitsubishi Tanabe Pharma Corp's edaravone Available on: https://www.accessdata.fda.gov/drugsatfda docs/nda/2017/209176Orig1s000PharmR.pdf

## Claims

1. Edaravone or a pharmaceutically acceptable salt thereof for use in the treatment of an oxidative stress-mediated neurodegenerative disorder, wherein the treatment comprises orally or gastrically administering any of them to human subjects in need thereof, once daily in the morning, at a daily dose ranging from 80 to 120 mg, during an uninterrupted period of at least 1 month, wherein the human subjects have fasted for at least 2 hours before administration.

2. Edaravone or a pharmaceutically acceptable salt thereof for use according to claim 1, wherein edaravone or a pharmaceutically acceptable salt thereof is administered at a daily dose ranging from 90 to 110 mg.

3. Edaravone or a pharmaceutically acceptable salt thereof for use according to any of the claims 1-2, wherein edaravone or a pharmaceutically acceptable salt thereof is administered during an uninterrupted period of at least 3 months.

4. Edaravone or a pharmaceutically acceptable salt thereof for use according to any of the claims 1-3, wherein the oxidative stress-mediated neurodegenerative disorder is Amyotrophic Lateral Sclerosis.

5. Edaravone or a pharmaceutically acceptable salt thereof for use according to claim 4, wherein the human subjects have a progression of the disease such that the total score variation of the Revised Amyotrophic Lateral Sclerosis Functional Rating Scale (ALSFRS-R) is lower than 1 point per month.

6. Edaravone or a pharmaceutically acceptable salt thereof for initial use according to any of the claims 4-5, wherein the human subjects have been diagnosed with Amyotrophic Lateral Sclerosis with an onset of symptoms of less than 24 months, more particularly of more than 1 month.

7. Edaravone or a pharmaceutically acceptable salt thereof for use according to any of the claims 4-6, wherein the human subjects have been diagnosed with definite ALS or Probable ALS based on EI Escorial revised criteria.

8. Edaravone or a pharmaceutically acceptable salt thereof for use according to any of the claims 1-7, wherein edaravone or a pharmaceutically acceptable salt thereof is administered in the form of a pharmaceutical composition which comprises a therapeutically effective amount of edaravone or a pharmaceutically acceptable salt thereof, together with one or more pharmaceutically acceptable excipients.

9. Edaravone or a pharmaceutically acceptable salt thereof for use according to claim 8, wherein the pharmaceutical composition is a liquid formulation selected from a solution and a suspension.

10. Edaravone or a pharmaceutically acceptable salt thereof for use according to claim 9, wherein the liquid formulation is a solution having a pH in the range from 6.0 to 9.0.

11. Edaravone or a pharmaceutically acceptable salt thereof for use according to claim 10, wherein the solution comprises:
a) from 0.05 to 3% by weight of edaravone or a pharmaceutically acceptable salt thereof, particularly edaravone;
b) from 0.1 to 6% by weight of a filler, particularly mannitol;
c) from 0.1 to 2% by weight of a pH adjusting agent, particularly disodium hydrogen phosphate;
d) from 0.001 to 0.5% by weight of a surfactant, particularly sodium lauryl sulfate;
wherein the weight percentages are expressed with respect to the total composition weight, and with the proviso that the sum of all the components of the composition is 100%.

12. Edaravone or a pharmaceutically acceptable salt thereof for use according to any of the claims 10-11, wherein the solution is prepared before its administration by mixing a solid particulate formulation comprising edaravone or a pharmaceutically acceptable salt thereof with a liquid and optionally one or more further pharmaceutically acceptable excipients.

13. Edaravone or a pharmaceutically acceptable salt thereof for use according to any of the claims 1-12, wherein 10 % or less of the human subjects show a shift from baseline from normal to low values of sensory nerve action potential amplitude (SNAP Amp) for the sural nerve; and
wherein 15 % or less of the human subjects show a shift from baseline from normal to low values in the sural-radial Amp ratio during the treatment;
wherein the normal values for the sensory nerve action potential amplitude (SNAP Amp) for the sural nerve are values higher than 8 µV for subjects with less than 40 years of age, higher than 5 µV for subjects with 40-60 years of age, and higher than 2 µV for subjects with more than 60 years of age; and
the normal values for the sural-radial Amp ratio (SRAR) are values higher than 0.45 for subjects with 18-39 years, higher than 0.32 for subjects with 40-59 years and higher than 0.19 for subjects with 60 or more years of age.

14. Edaravone or a pharmaceutically acceptable salt thereof for use according to any of the claims 1-13, wherein human subjects are treated without vitamin B6 supplementation.

15. Edaravone or a pharmaceutically acceptable salt thereof for use according to any of the claims 1-14, wherein the plasma concentration of pyridoxal 5'-phosphate in the human subjects during the treatment equals or exceeds 12 ng/L.
